# EUROPEAN PATENT SPECIFICATION

(11) **EP 4 412 701 B1**
(45) Date of publication and mention of the grant of the patent: **12.08.2026**
(21) Application number: 22799895.2
(22) Date of filing: 30.09.2022
(51) Int. Cl.: A61N 1/05, A61N 1/375, A61N 1/372

(54) **INTRA-CARDIAC DEVICE**
INTRAKARDIALE VORRICHTUNG
DISPOSITIF INTRACARDIAQUE

(30) Priority: 05.10.2021 US 202163252188 P; 01.11.2021 EP 21205747
(43) Date of publication of application: 14.08.2024
(73) Proprietor: BIOTRONIK SE & Co. KG, 12359 Berlin (DE)
(72) Inventor: TAFF, Brian M., Portland, Oregon 97217 (US); MUESSIG, Dirk, West Linn, Oregon 97068 (US); WHITTINGTON, R. Hollis, Portland, Oregon 97202 (US); AUSTIN, Eric, Portland, Oregon 97221 (US); HUGHES, Devan, Tualatin, Oregon 97062 (US); MIDGETT, Madeline Anne, Portland, Oregon 97213 (US)
(74) Representative: Biotronik Corporate Services SE
(86) International application number: PCT/EP2022/077346
(87) International publication number: WO 2023/057341

(56) References cited:
- EP-A1- 3 033 141
- EP-A1- 3 292 884
- US-A1- 2011 270 339
- US-A1- 2013 116 740
- US-A1- 2016 175 583
- US-A1- 2017 209 688

## Description

The present description refers to an implantable intra-cardiac device, such as an implantable intra-cardiac pacemaker. The invention is set out in the appended claims.

Active or passive intracardiac devices, for example implantable intracardiac pacemakers (also known as leadless pacemakers), are well known miniaturized medical devices which are entirely implanted into a heart's chamber or atrium of a patient. Intra-cardiac pacemakers are used for patients who suffer from a bradycardia, that is if a heart that beats too slow to fulfil the physiological needs of the patient. Intracardiac devices apply electrical stimulation in the form of pulses to the heart in order to generate a physiologically appropriate heartrate (intra-cardiac pacemakers) and/or in the form of shocks for cardioversion or defibrillation in order to restore a more normal heart rhythm. Alternative or additional functions of intra-cardiac devices comprise providing other electrical or electromagnetic signals to the heart or its surrounding tissue, sensing electrical or electromagnetic signals or other physiological parameters of the heart and/or its surrounding tissue.

Intra-cardiac device fixing mechanisms in the field are currently only used for ventricular implantation. As intracardiac pacemakers use is expanded to dual-chamber applications, specialized atrial fixing methods are also needed. Atrial anatomy dictates safe and reliable fixing approaches. The right atrium lateral wall and appendage (pocket off of main cavity) is extremely thin and sparsely covered with pectinate muscle. In contrast, the septal and posterior right atrium walls are smooth without any pectinate muscles.

Document US 8,700,181 B2 discloses an intracardiac medical device for implantation into one atrium comprising electrodes formed as fixing mechanism similar to a pair of large diameter double helices with a positive deflection near a base at the distal end of the device.

For fixing the double helices are screwed into the heart's wall. The purpose of this shape is to ease implantation of the device but rendering unscrewing of the device very difficult due to its firm adhering to the wall. Further, the distal ends of the double helices may have serrated edges that prevent the device from unscrewing out of the heart's chamber wall. Such fixing mechanism seems not applicable to the thin atrial walls. Documents US 2013/116740; EP 3 292 884; EP 3 033 141 and US 2011/270339 describe further implantable leadless devices known from the prior art.

Accordingly, there is the need for implantable intracardiac devices for atrial use having a fixing mechanism / anchoring method that would ensure reliable electrode contact with the thin walled atrial tissue substrate, simple implantation and low manufacturing effort and costs.

The above problem is solved by the intra-cardiac leadless pacemaker defined in claim 1. The present teaching also encompasses a non-claimed method for fixing of an intracardiac device, for example the leadless pacemaker according to claim 1, at an anchoring region within the tissue of a patient's heart.

In particular, the method for fixing of an intracardiac device at an anchoring region within the tissue of a patient's heart comprises the following steps, wherein the intracardiac device comprises one electrode and at least two tines attached at a distal end of the intra-cardiac device adjacent the electrode, wherein each of the at least two tines has a distal end in form of a hook with the furthest distal end section pointing inwards, is pivotable outwards around a bending point at its proximal end and/or bendable and is preloaded inwards:
- from the final state, the intra-cardiac device is moved towards the anchoring region with the at least two tines facing forwards until the at least two tines touch the tissue at the anchoring region,
- then, the at least two tines are pressed against the tissue at the anchoring region such that the at least two tines are pivoted outwards and/or bent outwards, thereby the distal ends of the at least two tines penetrate into the tissue (intermediate state), and,
- afterwards, the pressure on the at least two tines against the tissue is removed, following which the at least two tines relax to a more unconstrained state (final state), thereby bunching or grabbing a wall segment of the tissue by the at least two tines and pulling this wall segment towards the electrode.

Further, in particular the intra-cardiac device comprises one electrode and at least two tines attached at a distal end of the intra-cardiac device adjacent the electrode, wherein each of the at least two tines has a distal end in form of a hook with the furthest distal end section of the tine pointing inwards, is pivotable outwards around a bending point at its proximal end and/or bendable, wherein the at least two tines are further adapted
- to pivoted outwards and/or bent outwards when the at least two tines are pressed against the tissue of a patient's heart at an anchoring region, thereby the distal ends of the at least two tines penetrate into the tissue, and
- to bunch a wall segment of the tissue and to pull this wall segment towards the electrode when the intra-cardiac device is moved a pre-defined distance back from the anchoring region.

According to the invention as defined in claim 1, the implantable intracardiac leadless pacemaker device inter alia comprises a plurality of inward facing tines - i.e. hooks/curvature naturally biased towards a longitudinal axis of the device. Upon contact with anatomy (i.e. the surface of the heart's tissue) the physical interaction with the pre-curved tine shape forces the tines to open against the preload, splaying the individual tines outwardly by bending and/or pivoting. When relaxed, i.e. the applied force is removed, following which the intra-cardiac device moves a pre-defined distance back from the anchoring region, the hooked tips of the splayed tines grab onto the thin wall tissue bunch and pack the wall inward and toward the electrode.

The intracardiac device and fixing method according to the present teaching provide a reliable fixing with a reliable mechanical and electrical contact of the electrode with the thin walled atrial tissue substrate because the grabbing and bunching of the substrate towards the electrode brings the tissue closer to the electrode. Further, the invention allows to shorten the tine length necessary for device anchoring, particularly as compared to outward facing curvature of tine geometries common to devices designed for implant in the ventricle that are available today in the market. It is additionally of advantage, that in an anchored state of the proposed intra-cardiac device, compared to the tine-based anchors in presently available leadless pacing systems, there is little to no chance that tine tips would face outwards from the device in an exposed manner - which is a potential benefit for installation of subsequent devices which could mechanically or electrically engage with outward facing tines causing unwanted wear and/or compromises to device performance. Rather the distal tips of the tines reliably penetrate into the tissue. Additionally, for thin tissue substrates, the inventive intra-cardiac device and its respective fixing method forms a proactive means to force tissue/device contact as compared to designs prevalent in the market which, in thin tissues can leave a "gap" between the electrode and the tissue. Accordingly, it may be possible to consider support for right atrial placements of leadless pacemakers as one embodiment of the intra-cardiac device. However, the proposed design also may be used for fixing an intracardiac device in the right ventricle.

The implantable intracardiac device, for example a leadless pacemaker, may comprise a cylindrical housing having the above-mentioned longitudinal axis and the electrode projecting from the distal end of the housing, wherein the electrode may be pin-shaped and extending into the direction of the longitudinal axis. Further, a header assembly may be arranged at and attached to the distal end of the housing of the intracardiac device such that the electrode projects through the header assembly, i.e. a respective through-going or complete opening of the header assembly. The opening may be a central opening. The cylindrical housing comprises the electronics module particularly having an integrated circuitry inclusive of a processor, an energy source (e.g. a battery or coil (for wireless charging)) and, if applicable, communication component such as an antenna. The integrated circuitry may comprise a storage unit for data storage or one or more built-in memory allocations for data storage. The processor may be adapted to process signals determined from the patient's body or received from the surrounding environment and/or to produce signals for treatment of the patient's heart. Such signals may comprise electrical stimulation in the form of pulses in order to generate a physiologically appropriate heartrate, e.g. by providing an anti-bradycardia or anti-tachycardia pacing, shocks for cardioversion or defibrillation in order to restore a more normal heart rhythm and/or other electrical or electromagnetic signals to the heart or its surrounding tissue. Such signals are transformed and transmitted by the electronics module and may be applied by the pin-shaped electrode to the heart or its surrounding tissue. The pin-shaped electrode is electrically connected to the electronics module and the energy source. The hermetically sealed housing may comprise biocompatible material some of which may embody electrically conducting materials, e.g. Titanium, and may function as another electrode.

In one embodiment the movement of the intra-cardiac device towards the anchoring region is a pure longitudinal movement and/or the movement of the intra-cardiac device back from the anchoring region is a pure longitudinal movement or a combined longitudinal and rotational movement (i.e. a helical movement). The pure longitudinal movement is easy to realize during implantation, the combined movement provides a better anchoring of the tines within the heart's tissue. For example, the rotation may comprise minimum 10 degrees and/or maximum 45 degrees. As described above, relaxation of the tines results in a longitudinal movement of the intracardiac device back from the anchoring region (as a pure longitudinal movement or a combined movement). Such movement may have a length of approximately 3 mm or less.

In one embodiment of the fixing method during pressing the at least two tines the intra-cardiac device is moved towards the anchoring region in order to press the tines against the heart's tissue in the anchoring region and to pivot and/or bend the tines outwards against the preload (pretension) directed inwards. Due to the pivoting and/or bending, the distal end section of the at least two tines move such that the distal tip of each of the at least two tines and at least a part of the distal end section penetrate into the heart's tissue at the anchoring region. It is noted that caused by the pivoting and/or bending an outer distance D of the distal sections of opposite tines (distance D determined perpendicular to the longitudinal axis) becomes greater and the end sections of different tines penetrate into tissue points which are further apart than the outer distance of the end sections in the initial state (which is a relaxed state) prior pressing the tines against the tissue. When the tines move back from the tissue after penetration, the tissue between the penetrated points is bunched and packed inwards by the back-pivoting of the tines due to the preload because the outer distance D of the distal sections of the opposite tines reduces. Thereby the enhanced mechanical and electrical contact of the electrode with the tissue is provided. Accordingly, the outer distance D of the distal sections two opposite tines in the final state may be greater than in the initial state, wherein the distance D in the initial state is, for example, 2 mm to 5 mm and the distance of the in the final state is, for example, 7 mm to12 mm.

In one embodiment, at the distal end of the intracardiac device the electrode is located at a central position, preferably at the longitudinal axis of the intra-cardiac device, and the proximal ends of the at least two tines are located at a circumference around the electrode. In one embodiment each one tine of a pair of two tines is accommodated opposite to the other one of the pair. Thereby a good distribution of forces within the heart's tissue is provided, particularly when 4 or more tines are employed as part of the anchoring design.

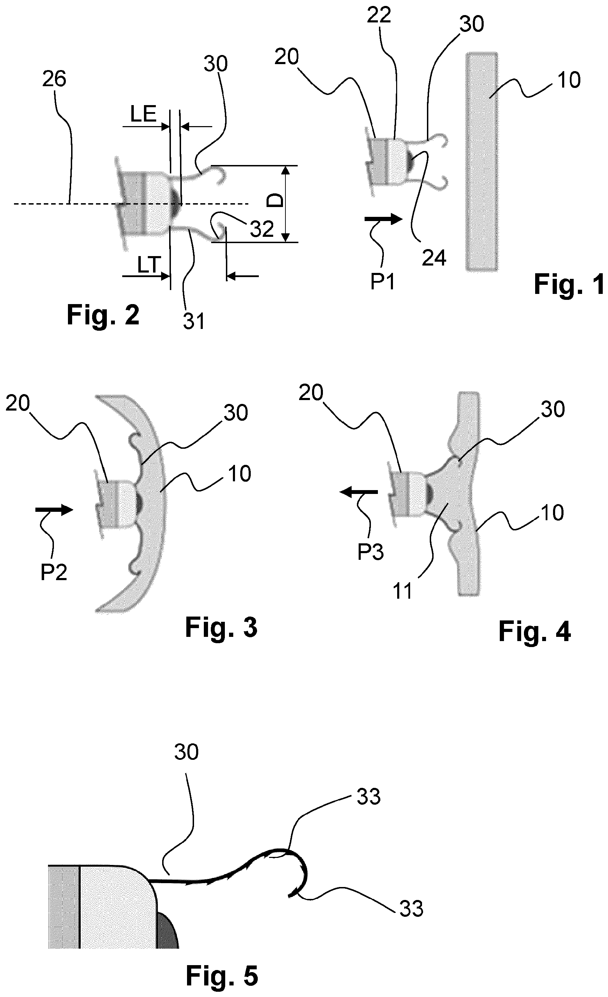

In one embodiment, the length ( e.g. as depicted in Figure 2 with the reference sign "LT") of the at least two tines is in the range of 2 mm to 10 mm and/or the exposed length of the electrode (the length with which the electrode projects from the housing or the header) is equal or less than 5 mm, wherein the each length is determined into the direction of the longitudinal axis of the intra-cardiac device and from the distal end of the header. The exposed length of the electrode is smaller than the length of the at least two tines.

Each tine has tines has a distal end in form of a hook (J-form) with at least one curve bending inwards so that the furthest distal end (section) of the tine points inwards. The at least one curve is located at the distal section of the tine. Each tine may be bendable in the region of the first curve, i.e. a proximal section of the tine.

In one embodiment, the diameter of the at least one curve at the distal end section of the at least two tines is in the range of 1 mm to 3 mm.

In one embodiment the distal end section of the at least two tines / the second curve is curved such that its respective furthest end (i.e. the distal tip of the tine) points towards the electrode. This embodiment provides a lower risk of injury upon initial tissue contact (as compared to tine-based leadless anchors on the market today) because in the initial state the furthest end is protected. However, when the pressing of the at least two tines against the tissue occurs and each tine is pivoted or bent, the furthest end of the tines opens and, due to this movement, the furthest distal end points increasingly toward the tissue, i.e. extends inclined to the longitudinal axis, providing a means for direct tissue engagement.

In one embodiment, the distal section of the at least two tines comprises one more barbs. In one embodiment, the distal section of the at least two tines comprises one barb being arranged at the furthest distal end section of the respective tine. In one embodiment, the distal section of the at least two tines comprises a plurality of barbs, wherein one barb of the plurality of barbs is arranged at the furthest distal end section of the respective tine

In one embodiment the at least two tines comprise a proximal section and a distal section, whereby the proximal section of the is straight and parallel to the longitudinal axis of the intra-cardiac device and the distal section is formed as a hook. This embodiment is particular advantageous with respect to a small catheter profile. Furthermore, the straight proximal section ensures a direct approach of the tines to the tissue, which eases the splaying/outward bending of the tines when the tines are pressed against the tissue.

The present invention will now be described in further detail with reference to the accompanying schematic drawing, wherein
- Fig. 1: shows a distal section of a first embodiment of the intracardiac device according to the present teaching in a side view in an initial state of the fixing method,
- Fig. 2: depicts an enlarged side view of the distal section of the intracardiac device shown in Fig. 1 in its initial state,
- Fig. 3: shows the distal section of the embodiment of Fig. 1 in an intermediate state,
- Fig. 4: shows the distal section of the embodiment of Fig. 1 in a final state, and
- Fig.5: shows an enlarged view of a distal section of another embodiment of the intracardiac device according to the the present teaching.

Fig. 1 illustrates a distal end of a first embodiment of a fixing method of an implantable intra-cardiac device, e.g. a leadless pacemaker, in an initial state prior fixing of the device within the heart's tissue of a patient. The device is to be fixed in an atrial wall 10 of the patient's heart,

The intra-cardiac device comprises a cylindrical housing 20. A header 22 and a tip electrode 24 are located at the distal end of the housing 20. The tip electrode 24 protrudes through the header 22 and from its distal end face. The length LE of the electrode (see Fig. 2) projecting from the distal end face of the header 22 into the direction of a longitudinal axis 26 is, for example, equal or less than 5 mm.

The housing 20 of the intracardiac device contains a battery and an electronic module comprising a processor and ensures hermetically sealing of these components. These components are electrically connected to the electrode 24 and provide the electrical stimulation of the heart or processing of electrical signals determined from the heart. Further, the housing 20 may contain components for communication such as an antenna.

There are two or more tines 30 projecting from the distal end of the header 22. In preferred embodiments, three, four or more than four tines may be provided. The electrode 24 is centered located at the longitudinal axis of the device. The tines 30 are located at a circumference around the longitudinal axis thereby surrounding the electrode 26.

Each tine 30 comprises a distal end 32 in form of a hook with the furthest distal end section of the tine pointing inwards. The distal end 32 is formed such that in the initial state shown in Fig. 1 the furthest end of this curve points into the direction of the electrode 24, which means that this end section is not perpendicular to the longitudinal axis 26 but inclined to this axis. The length LT of the tine 30 into longitudinal direction is greater than the length LE of the electrode 24, wherein the length LT is, for example, in the range of 2 mm to 10 mm.

The two tines 30 may be bent and/or pivoted outwards against a preloading directed inwards so that each tine 30 pivots around a point at the proximal end of the tine 30 and/or bends in its proximal section (first curve) as depicted in Fig. 3. When the pivoting and/or bending occurs, the outer distance D (see Fig. 2) of the distal sections of opposite tines 30 becomes greater (see Fig. 3 and 4).

Prior the first step of the fixing method shown in Fig. 1 the intra-cardiac device was minimally invasive implanted into the patient's heart using, for example, a catheter. Now, the device is in a distance with regard to an anchoring region of the, e.g., atrial wall 10 (see Fig. 1). The device is moved towards the anchoring region of the atrial wall 10 (see arrow P1 in Fig. 1) with the at least two tines facing forwards until the at least two tines 30 touch the tissue at the anchoring region. Then, the two or more tines 30 are pressed against the tissue at the anchoring region (see arrow P2 in Fig. 3) such that the two tines 30 are bent and/or pivoted outwards, thereby the distal ends of the at least two tines penetrate into the tissue. This is because due to the pivoting and/or bending the furthest tip of each tine 30 does not point inwards but away from the electrode 24 into the direction of the tissue (cf. Fig. 3). In the last step, the pressure on the tines 30 is removed, following which the tines 30 relax to an more unconstrained state, while the intra-cardiac device moves back from the anchoring region (e.g. equal to or less than 5 mm) by this relaxation of the tines 30 (see arrow P3 in Fig. 4), thereby bunching or grabbing a wall segment 11 of the tissue by the two tines 30 and pulling this wall segment 11 towards the electrode 24. A reliable mechanical and electrical contact is provided by this fixing method.

The material of the tines 30 is, for example nitinol.

The above explained tines 30 may be welded to the outer surface of the housing 20 or the header 22 of the intra-cardiac device. Other attachment mechanisms may comprise clamping of the base of the tines 30 within the header 22 or overmolding the base of the tines 30 within the header 22.

The above method is a reliable fixing method for an intra-cardiac device within an atrial wall of the heart or other thin walls of the heart. The method allows easy usage and is associated with low manufacturing costs.

## Claims

1. An intra-cardiac leadless pacemaker having a longitudinal axis and comprising one electrode (24) and at least two tines (30) attached at a distal end of the intra-cardiac pacemaker adjacent the electrode (24),
wherein each of the at least two tines (30) has a distal end in form of a hook with the furthest distal end section of the tine pointing inwards, i.e. towards the longitudinal axis of the pacemaker, is configured to be pivoted outwards around a bending point at its proximal end and/or bent,
wherein the at least two tines (30) are further adapted
- to pivot outwards and/or bend outwards when the at least two tines (30) are pressed against the tissue of a patient's heart at an anchoring region (arrow P2), thereby enabling the distal ends of the at least two tines to penetrate into the tissue, and
- to bunch a wall segment (11) of the tissue and to pull this wall segment (11) towards the electrode (24) when the intra-cardiac pacemaker is moved a pre-defined distance back from the anchoring region (arrow P3), and
whereby the tines comprise a proximal section and a distal section, whereby the proximal section is straight and parallel to the longitudinal axis of the intra-cardiac pacemaker when, in use, the intra-cardiac pacemaker is in a distance of the anchoring region and the distal section is formed as a hook.

2. The intra-cardiac leadless pacemaker according to claim 1, wherein, at the distal end of the intra-cardiac pacemaker, the electrode (24) is located at a central position and the proximal ends of the at least two tines (30) are located at a circumference around the electrode (24).

3. The intra-cardiac leadless pacemaker according to any of the claims 1 and 2, wherein the length of the at least two tines (30) is in the range of 2 mm to 10 mm and/or the length of the electrode (24) is equal to or less than 5 mm, wherein the each length is determined into the direction of a longitudinal axis (26) of the intracardiac device.

4. The intra-cardiac leadless pacemaker according to any of the claims 1 to 3, wherein the diameter of the distal end (32) of the at least two tines (30) is in the range of 1 mm to 3 mm.

5. The intra-cardiac leadless pacemaker according to any of the claims 1 to 4, wherein the distal end of the at least two tines (30) is curved such that its furthest distal end section points towards the electrode (24).

6. The intra-cardiac leadless pacemaker according to any of the claims 1 to 5, wherein at least the distal section of the at least two tines comprises one or more barbs.

## Patentansprüche

1. Intrakardialer, leitungsloser Schrittmacher mit einer longitudinalen Achse und umfassend eine Elektrode (24) sowie mindestens zwei Zinken (30), die an einem distalen Ende des intrakardialen Herzschrittmachers neben der Elektrode (24) angebracht sind, wobei jede der mindestens zwei Zinken (30) ein distales Ende in Form eines Hakens aufweist, wobei der am weitesten distale Endabschnitt der Zinke nach innen zeigt, d. h. in Richtung der longitudinalen Achse des Schrittmachers, und dazu konfiguriert ist, um einen Biegepunkt an ihrem proximalen Ende herum nach außen geschwenkt und/oder gebogen zu werden,
wobei die mindestens zwei Zinken (30) ferner dazu ausgelegt sind,
- nach außen zu schwenken und/oder sich nach außen zu biegen, wenn die mindestens zwei Zinken (30) an einem Verankerungsbereich gegen das Gewebe des Herzens eines Patienten gedrückt werden (Pfeil P2), wodurch die distalen Enden der mindestens zwei Zinken in das Gewebe eindringen können, und
- ein Wandsegment (11) des Gewebes zu bündeln und dieses Wandsegment (11) in Richtung der Elektrode (24) zu ziehen, wenn der intrakardiale Schrittmacher um einen vordefinierten Abstand vom Verankerungsbereich zurückbewegt wird (Pfeil P3), und wobei die Zinken einen proximalen Abschnitt und einen distalen Abschnitt umfassen, wobei der proximale Abschnitt gerade und parallel zur longitudinalen Achse des intrakardialen Schrittmachers verläuft, wenn sich der intrakardiale Schrittmacher im Einsatz in einem Abstand zum Verankerungsbereich befindet, und der distale Abschnitt als Haken ausgebildet ist.

2. Intrakardialer, leitungsloser Schrittmacher nach Anspruch 1, wobei sich am distalen Ende des intrakardialen Schrittmachers die Elektrode (24) in einer zentralen Position befindet und die proximalen Enden der mindestens zwei Zinken (30) auf einem Umfang um die Elektrode (24) herum angeordnet sind.

3. Intrakardialer, leitungsloser Schrittmacher nach einem der Ansprüche 1 und 2, wobei die Länge der mindestens zwei Zinken (30) im Bereich von 2 mm bis 10 mm liegt und/oder die Länge der Elektrode (24) gleich oder kleiner als 5 mm ist, wobei die jeweilige Länge in Richtung einer longitudinalen Achse (26) der intrakardialen Vorrichtung bestimmt wird.

4. Intrakardialer leitungsloser Schrittmacher nach einem der Ansprüche 1 bis 3, wobei der Durchmesser des distalen Endes (32) der mindestens zwei Zinken (30) im Bereich von 1 mm bis 3 mm liegt.

5. Intrakardialer, leitungsloser Schrittmacher nach einem der Ansprüche 1 bis 4, wobei das distale Ende der mindestens zwei Zinken (30) derart gebogen ist, dass sein am weitesten distaler Endabschnitt in Richtung der Elektrode (24) zeigt.

6. Intrakardialer, leitungsloser Schrittmacher nach einem der Ansprüche 1 bis 5, wobei der distale Abschnitt der mindestens zwei Zinken eine oder mehrere Widerhaken umfasst.

## Revendications

1. Stimulateur intra-cardiaque sans câbles ayant un axe longitudinal et comprenant une électrode (24) et au moins deux fourchons (30) attachés au niveau d'une extrémité distale du dispositif de stimulateur intra-cardiaque adjacent à l'électrode (24),
dans lequel chacun des au moins deux fourchons (30) a une extrémité distale sous forme de crochet avec la section d'extrémité distale la plus éloignée du fourchon pointant vers l'intérieur, c'est-à-dire vers l'axe longitudinal du stimulateur cardiaque, est conçu pour pivoter vers l'extérieur autour d'un point de pliure au niveau de son extrémité proximale et/ou plié,
dans lequel les au moins deux fourchons (30) sont en outre adaptés
- pour pivoter vers l'extérieur et/ou se plier vers l'extérieur lorsque les au moins deux fourchons (30) sont appuyés contre le tissu du cœur d'un patient au niveau d'une région d'ancrage (flèche P2), permettant ainsi aux extrémités distales des au moins deux fourchons de pénétrer dans le tissu, et
- d'agripper un segment de paroi (11) du tissu et de tirer ledit segment de paroi (11) vers l'électrode (24) lorsque le stimulateur intra-cardiaque est déplacé d'une distance prédéfinie en retour depuis la région d'ancrage (flèche P3), et
moyennant quoi les fourchons comprennent une section proximale et une section distale, moyennant quoi la section proximale est droite et parallèle à l'axe longitudinal du stimulateur intra-cardiaque lorsque, en cours d'utilisation, le stimulateur intra-cardiaque est à une distance de la région d'ancrage et la section distale est formée en crochet.

2. Stimulateur intra-cardiaque sans câbles selon la revendication 1, dans lequel, au niveau de l'extrémité distale du stimulateur intra-cardiaque, l'électrode (24) est située au niveau d'une position centrale et les extrémités proximales des au moins deux fourchons (30) sont situées au niveau d'une circonférence autour de l'électrode (24).

3. Stimulateur intra-cardiaque sans câbles selon l'une quelconque des revendications 1 et 2, dans lequel la longueur des au moins deux fourchons (30) est dans la plage de 2 mm à 10 mm et/ou la longueur de l'électrode (24) est inférieure ou égale à 5 mm, dans lequel chaque longueur est déterminée dans la direction d'un axe longitudinal (26) du dispositif intracardiaque.

4. Stimulateur intra-cardiaque sans câbles selon l'une quelconque des revendications 1 à 3, dans lequel le diamètre de l'extrémité distale (32) des au moins deux fourchons (30) est dans la plage de 1 mm à 3 mm.

5. Stimulateur intra-cardiaque sans câbles selon l'une quelconque des revendications 1 à 4, dans lequel l'extrémité distale des au moins deux fourchons (30) est incurvée de telle sorte que sa section d'extrémité distale la plus éloignée est orientée vers l'électrode (24).

6. Stimulateur intra-cardiaque sans câbles selon l'une quelconque des revendications 1 à 5, dans lequel au moins la section distale des au moins deux fourchons comprend un ou plusieurs ardillons.
